# EUROPEAN PATENT APPLICATION

(11) **EP 3 511 367 A1**
(43) Date of publication of application: **17.07.2019**
(21) Application number: 17848622.1
(22) Date of filing: 29.08.2017
(51) Int. Cl.: C08J 5/18, B32B 25/08

(54) **FILM, FILM ROLL, FILM STORAGE CONTAINER, AND METHOD FOR PROTECTING CONTAINER IN WHICH FILM IS USED**

(30) Priority: 09.09.2016 JP 2016176965; 07.07.2017 JP 2017134013
(71) Applicant: Asahi Kasei Pax Corporation, Tokyo 101-0054 (JP)
(72) Inventor: ISHIZUKA Hirokazu, Tokyo 101-8101 (JP); OHNO Toshiaki, Tokyo 101-8101 (JP)
(74) Representative: D Young & Co LLP
(86) International application number: PCT/JP2017/030997
(87) International publication number: WO 2018/047682

(57) **Abstract**

Disclosed is a film adherable to polystyrene on at least one surface thereof.

## Description

### TECHNICAL FIELD

This disclosure relates to a film, a film roll, a film storage body, and a method of protecting a container by using a film. The film disclosed herein is suitably usable, for example, in the field of biology and biotechnology, in particular, for covering containers, chemical bottles, and the like used for cell culture, inspection, and the like.

### BACKGROUND

Culture plates, dishes, and flasks are widely used for culturing cells, spheroids, tissues, and the like in the field of biology and biotechnology. In particular, when a culture plate is used, cultures such as cells and culture media are usually added to the wells of the culture plate, the wells are covered with an adhesive film, and then the culture plate is placed in a cell culture vessel. In JP3063248U (PTL 1), it is attempted to suppress the contact between the culture solution in the wells and the adhesive by disposing the adhesive so as not to contact those portions of the adhesive film corresponding to the wells.

Also, in the field of biology and biotechnology, a medium used for culturing, a preservation solution for cryopreservation of cells, and various solvents for examining cells, in particular, among organic solvents, dimethylsulfoxide (DMSO), methanol, ethanol, and acetone are often used.

In the laboratory, medicines can be transferred to a container such as a culture plate and stored with the mouth covered with a film, or temporarily stored with the mouth covered with a sealing film instead of a chemical bottle stopper. In general, PARAFILM® manufactured by BEMIS is frequently used as such a sealing film (PARAFILM is a registered trademark in Japan, other countries, or both).

However, since conventional sealing films such as "PARAFILM" are made of paraffin, they have poor resistance to organic solvents, and there is a need for sealing films having solvent resistance in the field of biotechnology and biotechnology.
On the other hand, a polyolefin-based stretch film not using an adhesive has been proposed (US5341557A [PTL 2]).

### CITATION LIST

### Patent Literature

PTL 1: JP3063248U
PTL 2: US5341557A

### SUMMARY

### (Technical Problem)

However, in the conventional plate cover film described in PTL 1, it is presumed that adhesion between the plate and the covering film is accomplished by an adhesive, and the contact between the culture solution in the wells and the adhesive is inevitable, which fact is problematic in that a part of the adhesive eluted to the culture solution may adversely affect the cultures such as cells.

In addition, the conventional film described in PTL 2 has poor adhesion to polystyrene containers frequently used in the field of biology and biotechnology, which poses a problem in practical use.

It would thus be helpful to provide a film which is excellent in adhesion to polystyrene containers and which enables culturing, transporting, or storing cells and the like.

### (Solution to Problem)

The present disclosure provides the following:
[1] A film adherable to polystyrene on at least one surface thereof.
   Specifically, a first aspect of the present disclosure provides the following.
[2] The film according to [1], having an adhesion strength to polystyrene of 0.1 to 2.0 N/25 mm on the at least one surface, and an elution amount from the at least one surface to ethyl acetate of 20 g/m² or less at 25 °C.
[3] The film according to [1] or [2], having an air permeability at 20 °C of 200 sec/100 cc or less.
   A second aspect of the present disclosure provides the following.
[4] The film according to [1], wherein the film contains a styrene elastomer, and the film has a heat of fusion (ΔH) of 5 to 45 J/g, an arithmetic mean roughness (Ra) of 0.7 µm or less on the at least one surface, and a thickness of 3 to 200 µm.
[5] A film roll comprising: a core tube; and the film as recited in [4] wound around the core tube.
[6] A film storage body for storing the film roll according to [5], comprising:
   an outlet port from which a film pulled out from the film roll is taken out.
   A third aspect of the present disclosure provides the following.
[7] A method of protecting a container containing contents by using a film, wherein the film comprises at least one surface which is an adhesion surface adherable to the container, the adhesion surface is formed by an adhesion layer which is made of an elastomer containing 50 % by weight or more of at least one of a styrene-conjugated diene block copolymer or a hydrogenated product thereof, the adhesion surface has an adhesion strength to polystyrene of 0.1 to 2.0 N/25 mm, and an elution amount from the adhesion surface to ethyl acetate is 20 g/m² or less at 25 °C.

### (Advantageous Effect)

According to the present disclosure, it is possible to provide a film which is excellent in adhesion to polystyrene containers and enables culturing, transporting, or storing cells and the like under good environment.

### BRIEF DESCRIPTION OF THE DRAWINGS

In the accompanying drawings:

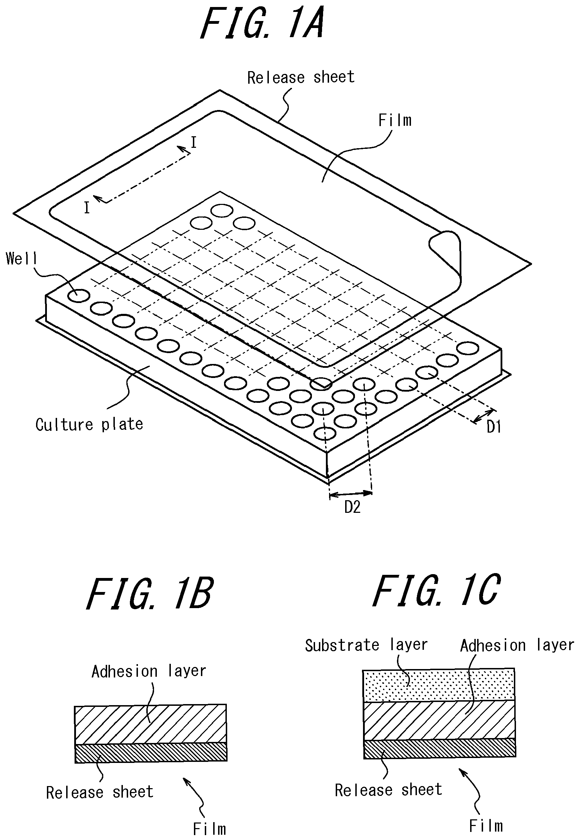

FIG. 1A is a perspective view illustrating an embodiment of the film disclosed herein together with a culture plate;
FIG. 1B illustrates a cross section in a plane along line I-I in FIG. 1A of an example of the film according to the embodiment; and
FIG. 1C illustrates a cross section in a plane along line I-I in FIG. 1A of another example of the film according to the embodiment.

### DETAILED DESCRIPTION

The following provides a detailed description of an embodiment of this disclosure (hereinafter, referred to as the "present embodiment"). However, the present disclosure is not limited to the following embodiment and may be implemented with various alterations that are within the essential scope thereof.

### (Film)

The film according to the present embodiment is a film adherable to polystyrene on at least one surface thereof.

The film according to the present embodiment is used for a culture plate and the like, and is particularly suitably used for covering some or all of the wells of a culture plate provided with the wells. In addition to the culture plate, the film according to the present embodiment may also be used for containers (e.g., dishes and flasks) used for cell culture and inspection, chemical bottles, or the like.

FIG. 1A is a perspective view illustrating an embodiment of the film disclosed herein together with a culture plate.

In a first aspect of the present disclosure, it is preferable that the film according to the present embodiment has an adhesion strength to polystyrene of 0.1 to 2.0 N/25 mm on the at least one surface, and an elution amount from the at least one surface to ethyl acetate of 20 g/m² or less at 25 °C.

The film according to the present embodiment has an adhesion strength to polystyrene within the above range and an elution amount to ethyl acetate within the above range at preferably 10 % or more, more preferably 30 % or more, and even more preferably 50 % or more of the at least one surface, and still more preferably has an adhesion strength to polystyrene within the above range over the entire surface (i.e., 100 %).

The film according to the present embodiment also includes those having an adhesion strength to polystyrene within the above range and an elution amount to ethyl acetate within the above range on both surfaces of the film.

In the film according to the present embodiment, it is preferable to provide a release sheet in a state of being in contact with the at least one surface.
Hereinbelow, a preferred embodiment of the film further comprising a release sheet will be described by way of example.

The film according to the present embodiment may be composed of a single layer or a laminate of a plurality of layers.
Hereinafter, one surface of the film to be affixed to the culture plate will be referred to as an adhesion surface, and a layer which forms the adhesion surface will be referred to as an adhesion layer.

FIG. 1B illustrates a cross section in a plane along line I-I in FIG. 1A of an example of the film according to the present embodiment.
An example of the film illustrated in FIG. 1B is formed by an adhesion layer and a release sheet laminated on the adhesion surface of the adhesion layer.

More specifically, an example of the film according to the present embodiment preferably has an adhesion strength to polystyrene of 0.1 to 2.0 N/25 mm on the adhesion surface.
Since most culture plates are made of polystyrene, the film according to the present embodiment needs to have adhesion strength with respect to a polystyrene plate, sheet, or film.
If the adhesion strength is less than 0.1 N/25 mm, the film tends to peel off due to vibration and impact when the culture plate containing the contents is transported with the film adhered to the culture plate and with the lid closed, and when the film surface faces upright or down, the film easily peels off and the contents easily leak out.
If the adhesion strength exceeds 2.0 N/25 mm, it is difficult to peel off the film affixed to the culture plate, and even if the film is peeled off, the contents are likely to be scattered.
Concretely, the adhesion strength to polystyrene can be measured by the method described further below in the EXAMPLES section.

The surface of the culture plate to which the film is affixed may be embossed or uneven depending on the manufacturer and is not necessarily flat. Thus, the adhesion strength is more preferably 0.2 N/25 mm or more.
Also, when handling the culture plate, the user often wears a rubber glove in a glove box or the like, and from the viewpoint of peelability, the adhesion strength is more preferably 1.5 N/25 mm or less.

Further, an example of the film according to the present embodiment preferably has an elution amount from the adhesion surface to ethyl acetate of 20 g/m² or less at 25 °C.

When the elution amount of the film from the side in contact with the culture plate (adhesion surface) to ethyl acetate exceeds 20 g/m² at 25 °C, the medium tends to be contaminated by the eluate, and the influence on the cells or the like could be no longer negligible.
In this respect, the medium and the cell cryopreservation solution contain water, inorganic salts, dimethylsulfoxide (DMSO), and amino acids such as glutamine and vitamins as nutrients. Since some of these components are prepared by a trace amount of formulation, foreign substances may have non-negligible effects on the cells.
Although ethyl acetate is not contained in the components of ordinary culture media or cell cryopreservation solutions, in evaluating the amount of substances that can be eluted from the film according to the present embodiment, it is preferable to use a medium in which the components constituting an adhesive or resin that can be contained in the film are more easily eluted from the perspective of judging whether or not culture in a favorable environment is made available or not. For this reason, ethyl acetate is adopted here.
The film according to the present embodiment has an elution amount to ethyl acetate of more preferably 10 g/m² or less, and further preferably 5 g/m² or less.
The elution amount to ethyl acetate may be specifically measured by the method described further below in the EXAMPLES section.

The following provides a description of a preferred aspect of the film according to the present embodiment in which the adhesion strength to polystyrene can be set to 0.1 to 2.0 N/25 mm and the elution amount from the adhesion surface to ethyl acetate can be 20 g/m² or less at 25 °C in at least a part of the adhesion surface.

That is, it is preferable that an example of the film according to the present embodiment has an arithmetic mean roughness (Ra) defined by JIS-B0601 of 0.01 to 0.70 µm on the side in contact with the culture plate (adhesion surface).
In an example of the film according to the present embodiment, it is preferable that the adhesion layer forming the surface in contact with the culture plate (adhesion surface) has a heat of fusion (ΔH) defined by JIS-K2122 of 1 to 45 J/g.

When the above-described Ra and/or ΔH is within the above range, an adhesion strength to polystyrene within the above range and an elution amount to ethyl acetate within the above range are easily achieved in the film according to the present embodiment.

The arithmetic mean roughness (Ra) of the adhesion surface is more preferably 0.01 to 0.2 µm.
The heat of fusion (ΔH) of the adhesion layer is more preferably 5 to 20 J/g.

The adhesion layer is preferably made of a resin.

Preferred resins used for the adhesion layer are a resin having a heat of fusion (ΔH) of 5 to 20 J/g, a low-density polyethylene having a density of 0.91 g/cm³ or less (including very-low-density polyethylene and ultra-low-density polyethylene), an ethylene-propylene copolymer (random or block copolymer), an ethylene-a olefin copolymer (random or block copolymer), a propylene-a olefin copolymer (random or block copolymer), an ethylene-propylene-a olefin copolymer (random or block copolymer), an acrylic acid-acrylic acid ester copolymer, an ethylene-styrene random copolymer, polyisoprene, natural rubber, a styrene-butadiene block copolymer, a hydrogenated product thereof, a styrene-isoprene block copolymer, polyurethane, and polysiloxane.
Among these resins, thermoplastic elastomers as defined in JIS K6200 are preferred. Specifically, an thermoplastic olefinic elastomer (TPO) which is composed of a blend of polyolefin and ordinary rubber and in which the rubber phase of the blend has no or almost no crosslinking point; a thermoplastic styrene elastomer (TPS) which is a copolymer of at least three blocks composed of styrene and specific diene, with two blocks (hard blocks) at both ends being polystyrene and inner block(s) (soft block(s)) being composed of polydiene or hydrogenated polydiene; and a thermoplastic polyurethane elastomer (TPU) made of block copolymers of hard blocks and soft blocks and having alternating hard segments and soft segments with the hard blocks being composed of urethane bond and the soft blocks being composed of ether, ester, or carbonate bond, or a mixture thereof, are more preferable. In particular, TPS is more preferable because it has excellent adhesion to polystyrene containers and other plastic containers.
These resins may be used alone or as a blend of two or more. In the case of a blend, the content of the resin in the adhesion layer is preferably 50 wt% or more, and more preferably 75 wt% or more. It is noted that in the case of a blend, the heat of fusion (ΔH) of the resin refers to a value in the blend.

The melt flow rate (MFR) (at 230 °C, 2.12 N) of the resin is preferably 1 to 100 g/10 min, and more preferably 2 to 50 g/10 min. It is noted that in the case of a blend of two or more resins or a mixture of additives such as an antistatic agent, a tackifier, and a flow control agent, the MFR refers to a value in the blend or the mixture.

The thickness of the adhesion layer is preferably 1 µm to 1 mm, and more preferably 3 µm to 200 µm. The thickness of the adhesion layer affects the adhesion to the plate, and is more preferably 30 µm to 150 µm.

Examples of the release sheet include: a sheet or film made of a polyolefin-based resin such as polypropylene (PP), high density polyethylene (HDPE), poly(4-methyl-1-pentene), or cyclic polyolefin (COC), a polyester resin such as polyethylene terephthalate (PET), polybutylene terephthalate (PBT), poly(trimethylene terephthalate) (PTT), or polycyclohexane dimethylene terephthalate (PCT), a resin such as polystyrene (PS), polyoxymethylene (POM), or polyamide (PA), paper, wood, non-woven or woven fabric, or the like; a metal or a foil thereof; and a product obtained by applying silicone coating, fluorine coating, surface roughening treatment, or the like thereto. From the viewpoints of handling properties and dust generation, a sheet or film made of a polyester resin or polystyrene (PS) is preferred.

The surface of the release sheet in contact with the adhesion layer of the film preferably has an arithmetic mean roughness (Ra) defined by JIS-B0601 of 0.70 µm or less.
Since the adhesion layer of the film is a resin or a resin composition called elastomer, and is flexible, irregularities on the surface of the release sheet in contact with the adhesion layer of the film may be transferred to the adhesion surface of the film depending on the conditions for transporting and storing the film, such as temperature, humidity, and time. When the Ra of the surface of the release sheet in contact with the adhesion layer is larger than 0.70 µm, the Ra of the surface (adhesion surface) of the film in contact with the culture plate may deviate from the preferable range.

Preferably, the film according to the present embodiment, with the release sheet removed, has an air permeability at 20 °C of 200 sec/100 cc or less when measured with a Gurley type densometer in accordance with JIS-P8117.
When the air permeability exceeds 200 sec/100 cc, in the case of culturing, transporting, and storing living cells at room temperature, it is concerned that the oxygen concentration in the wells will be reduced and the cells will die.
For a microplate having a larger number of wells than 96 wells, it is more preferable to use a film with even better gas permeability, in which the air permeability is 100 sec/100 cc or less, and more preferably 60 sec/100 cc or less. The oxygen permeability of a film having an air permeability of about 60 sec/100 cc corresponds to about 10⁷ cc/m² · day · atm.
The air permeability is also preferably 0.1 sec/100 cc or more.
When the air permeability is less than 0.1 sec/100 cc, the film has high breathability, and depending on the conditions such as temperature, humidity, and storage period, it is concerned that the components of the medium volatilize from the well through the film and the cells die out. The air permeability is more preferably 10 sec/100 cc or more.

In the present embodiment, in order to provide the film with air permeability as described above, the following approaches are possible: (i) using a material having high breathability, such as polyurethane or polysiloxane, for the adhesion layer, the substrate layer, and the like; (ii) using a so-called microporous membrane obtained by forming a layer in which inorganic materials and solvents are dispersed and removed so as to provide breathability; and (iii) subjecting the film to perforation processing. Among these, from the viewpoint of cost and internal visibility, perforation processing is preferable.

In the film according to the present embodiment, as described above, a plurality of holes may be provided by perforation processing.
It is preferable that the holes are uniformly provided in the plane of the film. The shape of the holes is not particularly limited, and may be round, square, elliptical, slit-like, or the like.
To achieve the above-described air permeability, it is preferable that the hole diameter is 0.1 to 500 µm and the interval between holes (hereinafter referred to as the "hole pitch") is 0.1 to 6.5 mm. The hole diameter is more preferably 0.5 to 100 µm, and even more preferably 1 to 30 µm. The hole pitch is more preferably 0.3 to 4 mm, even more preferably 0.5 to 3 mm, assuming a microplate having a larger number of wells than 96 wells.
The hole diameter refers to the diameter of the inscribed circle of the opening.
The hole diameter and the hole pitch may be appropriately adjusted so as to be suitable for the cells to be cultured. In the case of providing at least one hole for each well in a 96-well culture plate provided with wells at equal intervals in the longitudinal and transverse directions, for example, perforation processing may be performed with the hole pitch being a half (for example, 4.6 mm) of a distance D1 (for example, 9.2 mm, as in FIG. 1) between the centers of two adjacent wells, or with the hole pitch being a half (for example, 6.5 mm) of a distance D2 (for example, 13 mm, as in FIG. 1) between the centers of two wells shifted by one row in the longitudinal and transverse directions.
For perforation processing on such small holes, it is preferable to apply a technique, as described in JP2014237810A, that allows holes to be formed at very small and uniform pitches through the sheet to be subjected to perforation processing by passing the sheet through two rolls equipped with spiral protruding blades on the roll surfaces.

The film according to the present embodiment may be sterilized by, for example, EOG gas sterilization, ultraviolet sterilization, electron beam sterilization, or gamma radiation sterilization.

FIG. 1C illustrates a cross section in a plane along line I-I in FIG. 1A of another example of the film according to the present embodiment.
The film of another example shown in FIG. 1C comprises an adhesion layer, a release sheet laminated on the adhesion surface of the adhesion layer, and a substrate layer laminated on a surface opposite to the adhesion surface of the adhesion layer.

The adhesion layer and the release sheet in another example of the film according to the present embodiment may be the same as those in the above-described example of the film according to the present embodiment.

Examples of the resin used for the substrate layer include: a sheet or film made of a polyolefin-based resin such as polypropylene (PP), high density polyethylene (HDPE), poly(4-methyl-1-pentene), or cyclic polyolefin (COC), a polyester resin such as polyethylene terephthalate (PET), polybutylene terephthalate (PBT), poly(trimethylene terephthalate) (PTT), or polycyclohexane dimethylene terephthalate (PCT), a resin such as polystyrene (PS), polyoxymethylene (POM), polyamide (PA), polyurethane, or polysiloxane, paper, wood, non-woven or woven fabric, or the like; and a metal or a foil thereof. Among these, in particular, from the viewpoints of enabling observation inside the wells and providing sturdiness and high transparency, a sheet or film made of a polyester resin or polystyrene (PS) is preferred.

The thickness of the substrate layer is preferably 1 µm to 1 mm, and more preferably 5 µm to 0.5 mm.

The film according to the present embodiment may have other layers besides the aforementioned adhesion layer, substrate layer, and release sheet.

The culture plate used in the present embodiment may be a culture plate provided with one or more (for example, 6, 24, 96, 384, and so on) wells on its upper surface.
From the viewpoint of enhancing the adhesion between the culture plate and the film according to the present embodiment, it is preferable that the portions of the culture plate other than the openings of the wells on the upper surface are located on the same plane.

The plan view shape of the film for the culture plate according to the present embodiment is not particularly limited, and may be circular, elliptical, rectangular, or the like. In general, it may be a rectangular shape. The rectangular shape may be rounded at the corners.

The material forming at least the upper surface of the culture plate and the well surface is preferably a resin composition, and preferably contains a resin and, if necessary, other additives.
Examples of the resin include polystyrene, polyethylene, polypropylene, polyethylene terephthalate (PET), polybutene, polycarbonate, and poly(4-methyl-1-pentene).

In a second aspect of the present disclosure, it is preferable that the film according to the present embodiment contains a styrene elastomer, and has a heat of fusion (ΔH) of 5 to 45 J/g, an arithmetic mean roughness (Ra) of at least one surface of 0.7 µm or less, and a thickness of 3 to 200 µm.

The film according to the present embodiment has a heat of fusion (ΔH) of preferably 5 to 45 J/g, more preferably 5 to 30 J/g, and even more preferably 5 to 20 J/g. When ΔH is 5 to 45 J/g, the degree of crystallinity of the resin contained in the film is moderate, and the resistance to organic solvents (in particular, four organic solvents of DMSO, methanol, ethanol, and acetone) and the adhesion to the container (in particular, a polystyrene container) can be compatible with each other. Further, when adhesion at low temperature (e.g., -80 °C) is required, ΔH is preferably 20 J/g or less. The heat of fusion can be adjusted by, for example, the resin composition of the film, the melting temperature at the time of production, the extrusion temperature, and the cooling temperature.
It is noted here that the heat of fusion can be measured by the method in accordance with JIS K2122 described further below in the EXAMPLES section.

The film according to the present embodiment preferably has an arithmetic mean roughness (Ra) of 0.7 µm or less, and more preferably 0.2 µm or less, on at least one surface. When the arithmetic mean roughness is 0.7 µm or less, the adhesion to the container is excellent.
In the film according to the present embodiment, it is preferable that Ra is within the above range on one surface (in particular, the surface in contact with the container), yet Ra may be in the above range on both surfaces. In the film according to the present embodiment, it is preferable that the Ra on one surface on the side in contact with the container is not more than 0.7 µm and the Ra on the other surface is not less than 0.7 µm (for example, 0.7 µm or more and 2 µm or less) from the viewpoint that the film exhibits excellent adhesion to the container, does not wrinkle when rolled into a roll shape because blocking between films is suppressed at the time of winding a film into a roll shape, and can be pulled out lightly from the roll shape.
The arithmetic mean roughness (Ra) on the two surfaces may be the same or different. In addition, the arithmetic mean roughness (Ra) can be measured by the method in accordance with JIS B0601 described further below in the EXAMPLES section.

The film according to the present embodiment preferably has a thickness of 3 to 200 µm, and more preferably 20 to 100 µm. When the thickness is 3 to 200 µm, the handling property is improved and the film easily follows the shape of the container such that the adhesion to the container can be compatible. In the case of a film in which other layers are laminated as described later, the value of the above-described thickness refers to the thickness of the styrene elastomer layer, and the thickness of the entire laminate film is preferably 20 to 210 µm.

In the film according to the present embodiment, from the viewpoint of achieving both the adhesion to a container made of styrene and the resistance to organic solvents (in particular, four organic solvents of DMSO, methanol, ethanol, and acetone) at a higher level with a single-layer film, it is preferable that the thickness and the ΔH fall within the range bounded by points A (20, 20), B (50, 5), C (200, 5), D (200, 20), and E (20, 45) when the horizontal axis (x axis) is the thickness and the vertical axis (y axis) is ΔH.

From the viewpoints of handling properties and adhesion to the container, it is preferable that the film according to the present embodiment has a rupture strength of 8 to 25 MPa, and more preferably 10 to 20 MPa, when measured at a tension speed of 500 mm/min in accordance with JIS K6251.
In addition, the elongation at break is preferably 500 % to 1500 %, and more preferably 700 % to 100 %, when measured at a tensile speed of 500 mm/min in accordance with JIS K6251.

From the viewpoint of solvent resistance, it is preferable that all of the elution amounts to DMSO, methanol, ethanol, and acetone are less than 0.2 g/m², and more preferably less than 0.1 g/m².
The above-described elution amounts can be measured by the method described further below in the EXAMPLES section.

Next, a preferred aspect will be described in which the film according to the present embodiment may contain a styrene elastomer, and may have a heat of fusion (ΔH) of 5 to 45 J/g, an arithmetic mean roughness (Ra) of 0.7 µm or less on at least one surface, and a thickness of 3 to 200 µm.

The film according to the present embodiment is preferably a single-layer film, and may be a laminate film. In the case of the film according to the present embodiment being a laminate film, it is preferable that a surface layer including at least one surface of the laminate film contains a styrene elastomer, and the surface has an arithmetic mean roughness of 0.7 µm or less. In the case of a laminate film, the heat of fusion and the thickness refer to the measured values in the layer containing a styrene elastomer. It is also preferable that the rupture strength and the elongation at break of the laminate film are within the above-described ranges.

Examples of the styrene elastomer include a styrene thermoplastic elastomer defined by JIS K6200 which is a copolymer of at least three blocks composed of styrene and specific diene, with two blocks (hard blocks) at both ends being polystyrene and inner block(s) (soft block(s)) being composed of polydiene or hydrogenated polydiene.
Specific examples thereof include: a block copolymer of styrene and butadiene (TPS-SBS); polystyrene-poly(ethylene-butylene)-polystyrene (TPS-SEBS) which is a block copolymer of styrene and butadiene and which is obtained by hydrogenating soft blocks in which cis-1,4-butadiene units and 1,2-butadiene units are mixed; polystyrene-poly(ethylene-propylene)-polystyrene (TPS-SEPS) which is obtained by hydrogenating soft blocks in which most (for example, 90 mass% or more with respect to the total mass of the copolymer) of a block copolymer of styrene and isoprene is composed of 1,4-isoprene units; and a block copolymer of styrene and isoprene (SIS).
These styrene elastomers may be used alone or as a blend of two or more.

The film according to the present embodiment may contain another resin in combination with the styrene elastomer.
Examples of the other resin include polyethylene, polypropylene, ethylene-based copolymers, propylene-based copolymers, polyisoprene, natural rubber, polyolefin-based elastomer, and the like.
When two or more resins are contained, the heat of fusion (ΔH) refers to the value in the blend.

Optionally, a stabilizer such as a heat stabilizer or a weathering agent, a colorant such as a pigment or a dye, a lubricant, a crosslinking agent, a crosslinking aid, an anti-blocking agent, an anti-fogging agent, a filler such as an organic or inorganic filler, or a flow control agent may be added to the film according to the present embodiment.

At least one surface of the film according to the present embodiment may be covered with a release sheet from the viewpoint that the surface is easily protected until use and it is easy to wind in a roll shape. The release sheet may be provided on one or both surfaces of the film according to the present embodiment. It is preferable that the release sheet is at least provided on at least one surface of the film according to the present embodiment that has an arithmetic mean roughness of 0.2 µm or less. By providing the release sheet, it becomes easy to cut the film to a predetermined size.

As the release sheet, the same release sheet as that in the first aspect of the present disclosure may be used.

### (Method of Producing the Film)

In the second aspect of the present disclosure, a method for producing the film according to the present embodiment may include, for example, melting and kneading a raw material containing the above-described styrene elastomer using an extruder or the like, and extruding it into a sheet shape or the like from a T-die or the like.

In the film according to the present embodiment, a method of controlling the arithmetic mean roughness on the surface may include, for example, transferring irregularities to the film by overlapping the film on a release sheet having irregularities, transferring irregularities by passing the film through rollers having irregularities, and so on.

In winding up the film, it is preferable to consider preventing the formation of horizontal stripes or wrinkles caused by steps at the start of winding when winding up a soft material such as a paper tube with cushion or steps. From the viewpoint of preventing dust generation, it is better to use a plastic tube (commonly called "placore") rather than a paper tube.

### (Use of Film)

In the second aspect of the present disclosure, a method of using the film according to the present embodiment may include, for example, placing contents in a container such as a culture plate having a plurality of wells, a culture dish, or a culture flask, and covering the opening of the container.

The container is preferably made of polystyrene, polyester (e.g., PET), polyolefin (e.g., PP), or glass, and more preferably of polystyrene. Examples of the container include a container used for culturing, cryopreservation, inspection, or the like of cells, cell tissues, or the like. Among these, the film according to the present embodiment is preferably used for a container for regenerative medicine since it is easy to sterilize and generates less dust. In particular, by using a plastic core for the core tube of the film roll or by using a plastic case for the film storage body, dust generation can be further suppressed.

The film according to the present embodiment may be sterilized by, for example, EOG gas sterilization, ultraviolet sterilization, electron beam sterilization, or gamma radiation sterilization. In addition, the film according to the present embodiment may be used in, for example, a laboratory (such as, in particular, in a clean bench, in a draft chamber, in a clean room, in a laboratory with a biosafety level of 1 or more, or in an animal laboratory).

Conventional films had insufficient adhesion to plastic containers in general, not limited to polystyrene containers. In contrast, the film according to the second aspect of the present disclosure contains a polystyrene-based elastomer and has specific physical properties, and it can provide excellent adhesion to a variety of plastic containers, not limited to polystyrene containers (e.g., polyester containers and polyolefin containers).

### (Film Roll)

In the second aspect of the present disclosure, it is preferable that the film roll according to the present embodiment comprises a core tube and the above-described film wound around the core tube.

As the core tube, for example, a paper, plastic, or metal core tube can be used, yet it is preferable to use a lightweight plastic core tube with less dust generation. The core tube may be a hollow cylindrical object or a non-hollow cylindrical object. Also, the diameter of the core tube is not limited, and may be, for example, 1 to 6 inches.

The film roll according to the present embodiment may be produced by, for example, winding the above-described film on the core tube.

### (Film Storage Body)

In the second aspect of the present disclosure, it is preferable that the film storage body according to the present embodiment contains the film roll and comprises an outlet port from which the film pulled out from the film roll is taken out. The film storage body may be, for example, a storage unit in which the film roll is accommodated in, for example, a box such as a paper, wooden, metal, or resin box.

By making the film roll freely rotatable inside the storage body, it is possible to take out the film as needed.

Further, the film storage body according to the present embodiment may be provided with means for cutting the film pulled out from the core tube. This enables easy cutting of the pulled-out film as needed. As the cutting means, a slide cutter may be used in addition to a saw blade made of metal, paper, plastic, or the like. Alternatively, the pulled-out film may be cut off with scissors or the like without providing the cutting means.

It is preferable that the film according to the present embodiment is subjected to sterilization treatment either at the stage of formation of the film or the film roll, or at the stage of storage of the film roll in the film storage body. Examples of sterilization include radiation sterilization such as electron beam and gamma ray sterilization, ethylene oxide gas (EOG) sterilization, and autoclave sterilization, yet radiation sterilization is preferable in consideration of concern for a change in physical properties of the film and for residual gas.

### (Method of Protecting the Container Containing Contents using the Film)

As a third aspect of the present disclosure, in a method of protecting a container containing contents using the film according to the present embodiment, the film comprises at least one surface which is an adhesion surface adherable to the container, the adhesion surface is formed by a adhesion layer which is made of an elastomer containing 50 wt% or more of a styrene-conjugated diene block copolymer and/or a hydrogenated product thereof, the adhesion surface has an adhesion strength to polystyrene of 0.1 to 2.0 N/25 mm, and an elution amount from the adhesion surface to ethyl acetate is 20 g/m² or less at 25 °C.

Examples of the container according to the present embodiment include the above-mentioned culture plate, culture dish, and culture flask in which the film in the first and second aspects of the present disclosure is suitably usable. In particular, the method according to the present embodiment is suitably used for a multi-well plate such as a 96-well culture plate or a microplate having a larger number of wells than 96 wells. Examples of the contents of the container according to the present embodiment include a culture solution, a cell cryopreservation solution, a cell, a spheroid, and a tissue.

The elastomer constituting the adhesion layer of the film according to the present embodiment contains a styrene-conjugated diene block copolymer and/or a hydrogenated product thereof in an amount of 50 wt% or more, and from the viewpoint of adhesion, preferably 70 wt% or more, and more preferably 75 wt% or more.
As the elastomer according to the present embodiment, for example, the above-described elastomer contained in the film in the first and second aspects of the present disclosure is suitably usable.

The adhesion surface of the film according to the present embodiment has an adhesion strength to polystyrene of 0.1 to 2.0 N/25 mm, and preferably 0.2 to 1.5 N/25 mm from the same viewpoint as in the film in the first aspect of the present disclosure.
The adhesion strength to polystyrene can be measured by the method described further below in the EXAMPLES section.

Further, the elution amount from the adhesion surface of the film according to the present embodiment to ethyl acetate is 20 g/m² or less at 25 °C, and preferably 10 g/m² or less, and more preferably 5 g/m² or less, from the same viewpoint as in the film in the first aspect of the present disclosure. The elution amount to ethyl acetate can be specifically measured by the method described further below in the EXAMPLES section.

The film according to the present embodiment has an adhesion strength to polystyrene within the above range and an elution amount to ethyl acetate within the above range at preferably 10 % or more, more preferably 30 % or more, and even more preferably 50 % or more of the at least one surface, and still more preferably has an adhesion strength to polystyrene within the above range over the entire surface (i.e., 100 %).

The film according to the present embodiment also includes those having an adhesion strength to polystyrene within the above range and an elution amount to ethyl acetate within the above range on both surfaces of the film.

The film according to the present embodiment may be composed of a single layer or a laminate of a plurality of layers.

As a method of protecting the container according to the present embodiment, it is possible to perform the protection by affixing the adhesion surface of the film to the container so as to cover the opening of the container containing contents with a hand roller or the like.

### EXAMPLES

Examples of the present disclosure will now be described in detail below. However, the present disclosure is not limited to the disclosed examples.

### <Example A and Comparative Example A>

The materials used in Example A and Comparative Example A are as follows.

### (1) Resin for the Adhesion Layer

Elastomer 1: ZELAS® MC719 manufactured by Mitsubishi Chemical Corporation (ZELAS is a registered trademark in Japan, other countries, or both)
MFR (230 °C; 21.2 N) = 3 g/10 min, density = 0.89 g/cm³, melting point = 157 °C, ΔH = 15 J/g
Elastomer 2: ZELAS® 7055 manufactured by Mitsubishi Chemical Corporation
MFR (230 °C; 21.2 N) = 7 g/10 min, density = 0.89 g/cm³, melting point = 164 °C, ΔH = 61 J/g
Elastomer 3: Tuftec® H1221W manufactured by Asahi Kasei Corporation (Tuftec is a registered trademark in Japan, other countries, or both)
MFR (230 °C; 21.2 N) = 4.5 g/10 min, density = 0.89 g/cm³, melting point = 116 °C, ΔH = 0.5 J/g
Elastomer 4: Tuftec® L521W manufactured by Asahi Kasei Corporation MFR (230 °C; 21.2 N) = 15 g/10 min, density = 0.89 g/cm³, melting point = 103 °C, ΔH = 0.8 J/g
PP: WINTEC® WFX4M manufactured by Japan Polypropylene Corporation (WINTEC is a registered trademark in Japan, other countries, or both)
MFR (230 °C; 21.2 N) = 7 g/10 min, density = 0.9 g/cm³, melting point = 125 °C, ΔH = 72 J/g

### (2) Film for the Substrate Layer

### PET 1

A polyethylene terephthalate film manufactured by Toray Industries, Inc., having a thickness of 50 µm and subjected to corona treatment on the pasting surface: Lumirror® T60 (Lumirror is a registered trademark in Japan, other countries, or both) (3) Release Sheet

### Release sheet 1

A polyethylene terephthalate film manufactured by Toray Advanced Film Co., Ltd., having a thickness of 75 µm and subjected to silicone treatment on the pasting surface: Cerapeel® MFA (Cerapeel is a registered trademark in Japan, other countries, or both) The Ra measured by the method described below was 0.019 µm.

### Release sheet 2

A polyethylene terephthalate film manufactured by Toray Industries, Inc., having a thickness of 75 µm and subjected to matte processing; LUMIMAT® #300 TRES (LUMIMAT is a registered trademark in Japan, other countries, or both). The Ra measured by the method described below was 0.75 µm.

### (4) Adhesive

SK Dyne® E-4105 manufactured by Soken Chemical & Engineering Co., Ltd., which is an emulsion-type adhesive having an acrylic acid ester resin as a main component (SK Dyne is a registered trademark in Japan, other countries, or both)

### (5) Culture Plate

PrimeSurface® 96U MS-9096U (a 96-well plate) manufactured by Sumitomo Bakelite Co., Ltd. (PrimeSurface is a registered trademark in Japan, other countries, or both)

The analysis method and evaluation method used in Example A and Comparative Example A are as follows.

### (A) Adhesion Strength to Polystyrene

For the films prepared in Example A and Comparative Example A described below, the adhesion strength to polystyrene (in N/25 mm) was measured under the following conditions.
Polystyrene: an OPS film, GMCO, manufactured by Asahi Kasei Corporation, having a thickness of 50 µm
Measuring instrument: a universal testing machine, TENSILON RTC-1210A, manufactured by Orientec Co., Ltd.
Measurement environment: 23 °C, 50% RH
Specimen size: 25 mm wide × 100 mm long
Measurement conditions: 90° peeling (at a peeling speed of 50 mm/min)
Preprocessing: Before use, the surface of a polystyrene film was wiped with clean gauze, the pasting surface of the target film was overlapped thereon, the wiped surface and the target film were affixed to each other under a pressure of 0.1 MPa, and humidity conditioning was carried out for 30 minutes.

### (B) Elution Amount to Ethyl Acetate

For the films prepared in Example A and Comparative Example A described below, the elution amount (in g/m²) from the adhesion surface to ethyl acetate was measured under the following conditions.

After measuring and recording the weight of a glass petri dish manufactured by Tokyo Garasu Kikai Co., Ltd., having an inner diameter of 80 mm, with an electronic balance HM-200 model manufactured by A&D Co., Ltd., 40 mL of ethyl acetate (purity: 99.9 %) manufactured by Wako Pure Chemical Industries, Ltd. was added to the glass petri dish, the film was placed thereon, and the glass petri dish was lidded with another glass petri dish having the same diameter, and fixed together. This was then shaken at 25 °C with a shaker MK161, manufactured by Yamato Scientific Co., Ltd., at 60 rpm for 60 minutes. Thereafter, the lid was removed and the film was peeled off, and the petri dish containing the liquid was placed and dried in an air-blowing constant-temperature thermostat DK-400, manufactured by Yamato Scientific Co., Ltd., at 80 °C for 60 minutes. Then, the weight of the glass petri dish taken out from the thermostat was measured with the electronic balance, the difference between the measured weight and the weight of the glass petri dish itself measured in advance was calculated, and the result was converted into a square meter from the diameter of the glass petri dish to obtain the elution amount.

### (C) Arithmetic Mean Roughness (Ra)

For the films prepared in Example A and Comparative Example A described below, the arithmetic mean roughness (Ra) (in µm) of the adhesion surface was measured under the following conditions in accordance with JIS-B 0601. Measuring instrument: ACCRETECH SURFCOM 1400D-3DF manufactured byACCRETECH.
Measurement environment: 20 °C ± 1 °C, 60% RH
Stylus: tip = 2 µm, θ = 60°

### (D) Heat of Fusion (ΔH)

For the films prepared in Example A and Comparative Example A described below, the heat of fusion (ΔH) (in J/g) of the adhesion layer was measured under the following conditions in accordance with JIS-K2122.
Specimen: A 2.5 mg sample was taken from the adhesion layer using a cutter or the like and used for measurement.
Measuring instrument: a differential scanning calorimeter DSCQ-100 manufactured by TA Instruments Japan Inc.
Measurement conditions: The temperature was raised from -60 °C at 10 °C/min, held at 180 °C for 2 minutes, cooled to -60 °C at 10 °C/min, then raised again to 180 °C at 10 °C/min, and the heat of fusion at that point in time was determined.

### (E) Leakage Resistance

For the films prepared in Example A and Comparative Example A described below, the leakage resistance was evaluated under the following conditions. Evaluation Method: After placing 100 µL of purified water in each well of a culture plate and affixing the film to the plate with a pressure of 0.1 MPa using a hand roller, the plate was horizontally held and shaken by hand to visually check for liquid leaks (Evaluation 1). Samples with no liquid leakage were further stored for 5 days at -80 °C using an ultra-low temperature freezer CLN-32UW manufactured by Nippon Freezer Co., Ltd., and the peeling conditions were visually observed (Evaluation 2).

### Evaluation Criteria

- Excellent:: no peeling occurred even in Evaluation 2.
- Good:: no liquid leakage occurred in Evaluation 1.
- Fair:: in Evaluation 1, part of the film was peeled off and liquid leakage occurred.
- Poor:: the film was peeled off and liquid leakage occurred.

### (F) Peelability

For the films prepared in Example A and Comparative Example A described below, peelability was evaluated under the following conditions.
Evaluation Method: After placing 100 µL of purified water in each well of a culture plate and affixing the film to the plate with a pressure of 0.1 MPa using a hand roller, the film was peeled off by hand wearing ultra-thin nitrile gloves manufactured by Kawanishi Industry Co., Ltd. to observe the peeling conditions.

### Evaluation Criteria

Good: the film was peeled off from the plate without splashing of the liquid.
Fair: although the film was peeled off, a force was applied, and the liquid splashed.
Poor: the film could not be peeled off.

### (G) Elusion to Medium

For the films prepared in Example A and Comparative Example A described below, elution to the medium was evaluated under the following conditions.
Medium: a cryopreservation medium FM-1 manufactured by Kyokuto Pharmaceutical Industrial Co., Ltd.
Composition (in 1 L)
Eagle's MEM medium: 9.4 g
Methylcellulose: 2.0 g
Dimethylsulfoxide: 200 mL
L-Glutamine: 0.3 g
Sodium hydrogen carbonate: 1.0 g
Pure water: 800 mL
Evaluation Method: After placing 100 µL of the culture solution in each well of a culture plate, the film was affixed to the plate with a pressure of 0.1 MPa using a hand roller. Then, the culture solution was shaken such that the culture solution was adhered to the surface to which the film had been affixed, and the plate was allowed to stand at 25 °C for 7 days. After the lapse of time, a sample injection needle was inserted while the film was affixed, and the components of the culture solution in the wells were analyzed with a liquid chromatography mass spectrometer LCMS-8060 manufactured by Shimadzu Corporation.

### Evaluation Criteria

- Excellent:: the total amount of eluate was less than 10 µg/100 µL.
- Good:: the total amount of eluate was 10 µg/100 µL or more and less than 50 µg/100 µL.
- Fair:: the total amount of eluate was 50 µg/100 µL or more and less than 100 µg/100 µL.
- Poor:: the total amount of eluate was 100 µg/100 µL or more.

### (H) Hole Diameter and Hole Pitch

For the perforated films prepared in Example A and Comparative Example A described below, the hole diameter (in µm) and the hole pitch (in mm) were measured using a digital microscope VHX-1000 manufactured by Keyence Corporation and its measuring software. The measurement magnification was appropriately selected according to the hole diameter and the hole pitch.

### (I) Air permeability

For the films prepared in Example A and Comparative Example A described later, the air permeability (in sec/100 cc) was measured at 20 °C in accordance with JIS-P8117 by a densometer manufactured by Yasuda Seiki Seisakusho, Ltd.

### (J) Cell Spheroid Size and Cell Viability

Cell culture was carried out using the films prepared in Example A and Comparative Example A described later, and the cell spheroid size and the cell viability were calculated with a digital microscope VHX-1000 manufactured by Keyence Corporation.

### Other specifications are as listed in Table 1.

### (Test Example A)

Elastomer 1 was thermally melted using an extruder having a cylinder diameter (D) of 90 mm and L/D = 35, and extruded from a T-die having a width of 1000 mm such that the resin temperature became 280 °C and the thickness became 80 µm, to obtain an adhesion layer. A release sheet 1 was laminated on one surface of the adhesion layer through a casting roll having a surface temperature of 15 °C and a rubber back-up roll, and the laminate was taken off.

Then, the substrate layer was affixed to the surface of the adhesion layer of the laminate using a polyurethane-based adhesive, and this was cut into 79 mm × 120 mm (Example A1).

On the other hand, the adhesive was coated on one surface of the substrate layer with a bar coater so as to have a coating amount of 22 g/m² after drying, dried at 85 °C for 3 minutes, and left at room temperature for 5 minutes. Then, the release sheet 1 was overlapped on the other surface of the substrate layer, and the resultant was cut into 79 mm × 120 mm (Comparative Example A1).

Table 1 summarizes the evaluation results for Example A1, which was a film configured according to the present disclosure, and for Comparative Example A1, which used an adhesive imitating a conventional product.

**Table 1**

| | | | | | | Example A1 | Comparative Example A1 |
|---|---|---|---|---|---|---|---|
| Film | Layered structure | | | | - | Adhesion layer + Substrate layer | Substrate layer with adhesive |
| | Substrate layer | Resin | PET1 | Lumirror T60 | mass% | 100 | 100 |
| | | Thickness | | | µm | 50 | 50 |
| | Adhesion layer | Resin | Elastomer 1 | ZELAS MC719 | mass% | 100 | - |
| | | | Elastomer 2 | ZELAS 7055 | mass% | - | - |
| | | | Elastomer 3 | H1221W | mass% | - | - |
| | | | Elastomer 4 | L521W | mass% | - | - |
| | | | PP | WINTEC WFX4M | mass% | - | - |
| | | Thickness | | | µm | 80 | 22 |
| | Release sheet | | | | - | Sheet 1 | Sheet 1 |
| Evaluation | (A) Adhesion strength to polystyrene | | | | N/25mm | 0.9 | 2.1 |
| | (B) Elution amount to ethyl acetate | | | | g/m² | 2 | 22 |
| | (C) Arithmetic mean roughness (Ra) | | | | µm | 0.04 | 1.05 |
| | (D) Heat of fusion (ΔH) | | | | J/g | 15 | approx. 0 |
| | (E) Leakage resistance | | | | - | Excellent | Good |
| | (F) Peelability | | | | - | Good | Fair |
| | (G) Elusion to medium | | | | - | Excellent | Poor |

It can be seen from Table 1 that Example A1 was excellent in all of leak resistance, peelability, and low elution property. In contrast, Comparative Example A1 in which the adhesive (ΔH ≈ 0 J/g) eluted was markedly inferior in low elusion property, and in comparison with Example A1, it was inferior in terms of leak resistance at low temperature and peelability.

### (Test Example B)

The same operations as in Example A1 of Test Example A were repeated except that Example A2 used a composition in which PP and Elastomer 3 were melt blended as an adhesion layer at a weight ratio of 50:50 in a twin screw extruder, Example A3 used a composition in which PP and Elastomer 4 were melt blended as an adhesion layer at a weight ratio 25:75 in a twin screw extruder, and Comparative Example A2 used Elastomer 2 as an adhesive layer.

The same operations as in Example A3 were repeated except that the release sheet 1 was changed to the release sheet 2 (in Comparative Example A3). The evaluation results for Examples A2 and A3 and Comparative Examples A2 and A3 are summarized in Table 2.

**Table 2**

| | | | | | | Example A2 | Example A3 | Comparative Example A2 | Comparative Example A3 |
|---|---|---|---|---|---|---|---|---|---|
| Film | Layered structure | | | | - | Adhesion layer + Substrate layer | Adhesion layer + Substrate layer | Adhesion layer + Substrate layer | Adhesion layer + Substrate layer |
| | Substrate layer | Resin | IPET1 | Lumirror T60 | mass% | 100 | 100 | 100 | 100 |
| | | Thickness | | | µm | 50 | 50 | 50 | 50 |
| | Adhesion layer | Resin | Elastomer 1 | ZELAS MC719 | mass% | - | - | - | - |
| | | | Elastomer 2 | ZELAS 7055 | mass% | - | | 100 | |
| | | | Elastomer 3 | H1221W | mass% | 50 | - | - | - |
| | | | Elastomer 4 | L521W | mass% | - | 75 | - | 75 |
| | | | PP | WINTEC WFX4M | mass% | 50 | 25 | - | 25 |
| | | Thickness | | | µm | 80 | 80 | 80 | 80 |
| | Release sheet | | | | - | Sheet 1 | Sheet 1 | Sheet 1 | Sheet 2 |
| Evaluation | (A) Adhesion strength to polystyrene | | | | N/25 mm | 0.2 | 0.5 | < 0.1 | < 0.1 |
| | (B) Elution amount to ethyl acetate | | | | g/m² | 1 | 2 | 1 | 2 |
| | (C) Arithmetic mean roughness (Ra) | | | | µm | 0.03 | 0.02 | 0.1 | 0.75 |
| | (D) Heat of fusion (ΔH) | | | | J/g | 40 | 16 | 61 | 16 |
| | (E) Leakage resistance | | | | - | Good | Excellent | Poor | Poor |
| | (F) Peelability | | | | - | Good | Good | Good | Good |
| | (G) Elusion to medium | | | | - | Excellent | Excellent | Excellent | Excellent |

Evaluation was good for Examples A2 and A3 which are films according to the present disclosure.
In contrast, in Comparative Example A2, the ΔH was as high as 61 J/g, the adhesion to polystyrene was weak, and thus the leak resistance was inferior. In Comparative Example A3, the Ra was as large as 0.75 µm, and similar to Comparative Example A2, the leak resistance was inferior.

### (Test Example C)

With reference to JP2014237810A, the film of Example A1 of Test Example A was passed through two rolls, each being a metal roll having a roll diameter of 55 mm and a length of 55 mm and engraved with a helical blade having a blade width of 10 µm at a pitch of 2 mm thereon, and holes having a diameter of 10 µm were provided at a pitch of 2 mm (Example A4). The air permeability of the film was 200 seconds/100 cc.

In this test example, the film of Comparative Example A1 of Test Example A and the lid (conventional example) sold as a set with the culture plate were also used.

As a medium, a product obtained by adding 10% fetal bovine serum (FBS) manufactured by Cosmo Bio Co., Ltd. and antibiotics to DMEM manufactured by Sigma-Aldrich Japan Inc. was used, and human fibroblasts were seeded on a plurality of culture plates, and left at room temperature for 2 days.

The culture plates under cell culture thus prepared were divided into three groups, in one of which group the culture plate was covered with the film of Example A4 and in another group the culture plate was lidded with the film of Comparative Example A1. The above two groups were placed in a conveying container adjusted to room temperature and left for 24 hours. The remaining one group was covered with a conventional lid and subjected to cell culture in a CO₂ incubator for 24 hours (control condition).

The observation of the cell clusters (spheroids) in these three groups with the above-described microscope revealed that those lidded with the conventional lid and those covered with the film of Example A4 similarly had a spheroid diameter of 700 µm, whereas for those lidded with the film of Comparative Example A1, the spheroid diameter was 750 µm. The cohesive force of spheroids was weak in Comparative Example A1 as compared with the conventional example and Example A4.

In addition, the cell viability of the cells was 97.6 % for those covered with a conventional lid, while 97.5 % for those lidded with the film of Example A4. Example A4 appeared to be in an environment in which the cells were somewhat hard to breathe, yet exhibited a cell viability comparable to that of the conventional example. Further, with respect to those lidded with the film of Example A4, an organ could be prepared without problems even with spheroids taken out from the culture plate after stored for another 1 day.

From the above, it was found that the film of the present embodiment can maintain the culture environment in the wells even with the film being affixed to the plate, and can be used for a series of steps, from culture, transportation, and storage.

<Example B, Comparative Example B, and Reference Example B> The materials used in Example B, Comparative Example B, and Reference Example B are as follows.

### (1) Resin

Elastomer 1: a mixture of a styrene elastomer and an olefin elastomer Zelas® MC719 manufactured by Mitsubishi Chemical Corporation (Zelas is a registered trademark in Japan, other countries, or both)
MFR (230 °C; 21.2 N) = 3 g/10 min, density = 0.89 g/cm³, melting point = 157 °C, ΔH = 15 J/g
Elastomer 2: olefin elastomer (ethylene-methyl methacrylate copolymer resin) Acryft® HW303-F manufactured by Sumitomo Chemical Co., Ltd. (Acryft is a registered trademark in Japan, other countries, or both)
Methyl methacrylate content = 18%, MFR (190 ° C; 21.2 N) = 7, melting point = 87 °C, ΔH = 18 J/g
Elastomer 3: styrene elastomer (SEBS)
Tuftec® H1221W manufactured by Asahi Kasei Corporation (Tuftec is a registered trademark in Japan, other countries, or both)
MFR (230 °C; 21.2 N) = 4.5 g/10 min, density = 0.89 g/cm³, melting point = 116 °C, ΔH = 0.5 J/g
Elastomer 4: styrene elastomer (SEBS)
Tuftec® L521W manufactured by Asahi Kasei Corporation
MFR (230 °C; 21.2 N) = 15 g/10 min, density = 0.89 g/cm³, melting point = 103 °C, ΔH = 0.8 J/g
PP: polypropylene resin
WINTEC® WFX4M manufactured by Japan Polypropylene Corporation (WINTEC is a registered trademark in Japan, other countries, or both)
MFR (230 °C; 21.2 N) = 7 g/10 min, density = 0.9 g/cm³, melting point = 125 °C, ΔH = 72 J/g

### (2) Release Sheet

A polyethylene terephthalate film manufactured by Toray Advanced Film Co., Ltd., having a thickness of 75 µm and subjected to silicone treatment on the pasting surface: Cerapeel® MFA (Cerapeel is a registered trademark in Japan, other countries, or both) The Ra measured by the method described below was 0.019 µm.

The analysis method and evaluation method used in Example B, Comparative Example B, and Reference Example B are as follows.

### (A) Adhesion

Water was filled in Styrol Schale NH-52 (diameter: 52 mm, height: 10 mm), which is a polystyrene petri dish manufactured by AS ONE Corporation. Each petri dish was covered with respective films obtained in Example B and Comparative Example B described later from above to the lower end of its side surface, and evaluated as "Good" if water did not leak when turned over and as "Poor" if water leaked.

Also, 50 cc of water was placed in a glass reagent bottle having a capacity of 50 mL, the mouth of the reagent bottle was covered with respective sample films, and those in which water did not leak when turned over were evaluated as "Good" and as "Poor" in which water leaked.

### (B) Solvent Resistance

The elution amount (in g/m²) to each solvent was measured for the films of Example B and Comparative Example B described later under the following conditions.

A glass flask with a stopper having internal volume of 250 mL was charged with 100 cc of four kinds of solvents, dimethylsulfoxide (DMSO), methanol, ethanol, and acetone.

Then, each sample film was cut into 20 mm × 50 mm, precisely weighed, placed in flasks containing the above solvents, and left at 23 °C for 30 minutes.

Then, each sample film was taken out from the solvents, left in an 80 °C atmosphere for 10 minutes to volatilize the solvents, the weight was precisely weighed, and the elution amount was determined from the weight difference before and after immersion in the solvents (g/m²).

The evaluation was scored as "Excellent" when the elution amount was less than 0.1 g/m², "Good" when the elution amount was 0.1 g/m² or more and less than 0.2 g/m², "Fair" when the elution amount was 0.2 g/m² or more and 0.4 g/m² or less, and "Poor" when the elution amount exceeded 0.4 g/m².

### (C) Heat of fusion (ΔH)

The heat of fusion (ΔH) (in J/g) of the adhesion layer was measured for the films prepared in Example B and Comparative Example B described below under the following conditions in accordance with JIS-K2122.
Specimen: About 5 mg was sampled from each sample film, precisely weighed, and used for measurement.
Measuring instrument: a differential scanning calorimeter DSCQ-100 manufactured by TA Instruments Japan Inc.
Measurement conditions: The temperature was raised from -60 °C at 10 °C/min, held at 180 °C for 2 minutes, cooled to -60 °C at 10 °C/min, then raised again to 180 °C at 10 °C/min, and the heat of fusion (in J/g) at that point in time was determined.

### (D) Arithmetic Mean Roughness (Ra)

The arithmetic mean roughness (Ra) (in µm) of the adhesion surface and the non-adhesion surface was measured for the films prepared in Example B and Comparative Example B described below under the following conditions in accordance with JIS-B 0601.
Measuring instrument: ACCRETECH SURFCOM 1400D-3DF manufactured byACCRETECH.
Measurement environment: 20 °C ± 1 °C, 60% RH
Stylus: tip = 2 µm, θ = 60°

### (Test Example A')

Elastomer 1 was thermally melted using an extruder having a cylinder diameter (D) of 90 mm and L/D = 35, and extruded from a T-die having a width of 1000 mm such that the resin temperature became 280 °C and the thickness became 50 µm, to obtain a film. A release sheet was laminated on one surface of the film through a casting roll having a surface temperature of 15 °C and a rubber back-up roll, and the laminate was taken off. Then, the release sheet was peeled off and wound up to obtain a single-layer film having a thickness of 50 µm. The Ra of the peeled surface (surface in contact with the container) was 0.04 µm, and the Ra of the non-peeled surface was 0.75 µm. The heat of fusion (ΔH) of the film was 15 J/g, and the arithmetic mean roughness (Ra) was 0.04 µm.

Further, the same operations as in Example B1 were repeated except that Elastomer 2 (olefin elastomer) was used instead of Elastomer 1 (styrene elastomer) used in Example B1, to obtain a conventional film (in Comparative Example B1). The thickness of the film was 50 µm, the heat of fusion (ΔH) was 18 J/g, and the arithmetic mean roughness (Ra) was 0.04 µm.

The results of evaluation of adhesion and solvent resistance are summarized in Table 3 for the film of Example B1, the film of Comparative Example B1 which is a conventional olefin elastomer film, and the film of PARAFILM® (PARAFILM is a registered trademark in Japan, other countries, or both) manufactured by BEMIS, which is a paraffin film conventionally used as a reference in physicochemical experiments (Reference Example B1), Saran Wrap® (Saran Wrap is a registered trademark in Japan, other countries, or both) which is a vinyl chloride-vinylidene chloride copolymer film manufactured by Asahi Kasei Corporation (Reference Example B2) and used as a substitute for a sealing film, and "Daiso Value Pack Food Wrap" (Reference Example B3) manufactured by Daiso Co., Ltd., which is a polyethylene-based resin.

**Table 3**

| | | | Example B1 | Comparative Example B1 | Reference Example B1 | Reference Example B2 | Reference Example B3 |
|---|---|---|---|---|---|---|---|
| Film | Composition | Elastomer 1 | 100 | - | - | - | - |
| | | Elastomer 2 | - | 100 | - | - | - |
| Evaluation | Thickness (µm) | | 50 | 50 | 130 | 10 | 10 |
| | (A) Adhesion | Glass | Good | Good | Good | Good | Good |
| | | PS | Good | Poor | Good | Poor | Poor |
| | (B) Solvent resistance | DMSO | Excellent | Excellent | Excellent | Fair | Excellent |
| | | Methanol | Excellent | Excellent | Excellent | Good | Good |
| | | Ethanol | Excellent | Good | Poor | Good | Excellent |
| | | Acetone | Excellent | Good | Poor | Poor | Excellent |

The film of Example B1 was excellent both in adhesion and solvent resistance. Handling was also satisfactory without problems. Further, the above-described polystyrene container was charged with water and covered with the film. The film was not peeled off immediately after the container was taken out after having been left at -80 °C for 3 days.

In contrast, the conventional film (Comparative Example B1) had poor adhesion to the polystyrene container although it had good solvent resistance. In addition, PARAFILM® manufactured by BEMIS (Reference Example B1), which has been used conventionally, was inferior in solvent resistance, Saran Wrap® manufactured by Asahi Kasei Corporation (Reference Example B2) and "Daiso Value Pack Food Wrap" manufactured by Daiso Co., Ltd. (Reference Example B3) were also inferior in adhesion to the polystyrene containers.

### (Test Example B')

The same operations as in Example B1 were repeated to obtain each film except that Elastomer 1 of Example B1 was replaced with a resin composition in which 50 wt% of Elastomer 3 and 50 wt% of PP were mixed (in Example B2), a resin composition in which 85 wt% of Elastomer 4 and 15 wt% of PP were mixed (in Example B3), 100 wt% of Elastomer 4 (in Comparative Example B2), or a resin composition in which 75 wt% of Elastomer 4 and 25 wt% of PP were mixed (in Comparative Example B3). However, the thickness of the film was 20 µm in Example B2, 120 µm in Example B3, 250 µm in Comparative Example B2, and 50 µm in Comparative Example B3. In Comparative Example B3, the release sheet was changed to one having Ra of 0.75 µm. The adhesion and solvent resistance of Example B2, Example B3, Comparative Example B2, and Comparative Example B3 were evaluated as described above. The results are summarized in Table 4.

**Table 4**

| | | | Example B1 | Example B2 | Example B3 | Comparative Example B2 | Comparative Example B3 |
|---|---|---|---|---|---|---|---|
| Film | Composition | Elastomer 1 | 100 | - | | - | - |
| | | Elastomer 3 | - | 50 | - | - | - |
| | | Elastomer 4 | | - | 85 | 100 | 75 |
| | | PP | - | 50 | 15 | - | 25 |
| Evaluation | Thickness (µm) | | 50 | 20 | 120 | 250 | 50 |
| | (C) Heat of fusion (J/g) | | 15 | 40 | 7 | 1 | 16 |
| | (D) Arithmetic mean roughness (µm) | Peeled surface | 0.04 | 0.03 | 0.02 | 0.02 | 0.75 |
| | | Non-peeled surface | 0.75 | 0.75 | 0.75 | 0.75 | 0.75 |
| | (A) Adhesion | Glass | Good | Good | Good | Poor | Poor |
| | | PS | Good | Good | Good | Poor | Poor |
| | (B) Solvent resistance | DMSO | Excellent | Excellent | Excellent | Good | Excellent |
| | | Methanol | Excellent | Excellent | Good | Poor | Excellent |
| | | Ethanol | Excellent | Excellent | Good | Poor | Excellent |
| | | Acetone | Excellent | Excellent | Good | Poor | Excellent |

In the case of a composition having a relatively high ΔH of 40 J/g as in Example B2, it is preferable to make the thickness relatively small, 20 µm, from the viewpoint of further improving the adhesion to the container. There was somewhat difficulty in handling due to thin thickness (practically no problem).
For Example B3, the composition had a relatively low ΔH of 7 J/g, in which excellent adhesion to the container was obtained, and the film was not peeled off immediately after the polystyrene container charged with water and covered with the film was taken out after having been left at -80 °C for 3 days. In contrast, Comparative Example B2 for which ΔH was 1 J/g, which is outside the range of the present disclosure, was inferior in solvent resistance.
Further, Comparative Example B3 for which Ra was 0.75 µm, which is outside the range of the present disclosure, was inferior in adhesion to the container.

From the above, it was found that the film according to the present embodiment exhibits adhesion not only to glass containers, but also to polystyrene culture plates, dishes, and flasks widely used in the field of biology/biotechnology for culturing cells, spheroids, tissues, and the like, and has resistance to organic solvents such as dimethyl sulfoxide (DMSO), methanol, ethanol, acetone, and the like.

### INDUSTRIAL APPLICABILITY

According to the present disclosure, adverse effects on the cultured cells can be minimized by reducing, in each step of culturing, cryopreservation, and inspection of cells (including spheroids), cell tissues, and the like, elution and dissolution into a medium of an aqueous solution containing amino acids such as glutamine and nutrients such as vitamins, a cell cryopreservation solution containing inorganic salts, dimethylsulfoxide (DMSO), and the like, a reagent such as methanol, ethanol, or acetone, and cells and the like can be cultured, transported, or stored even with the film being affixed to the plate or the like. Furthermore, according to the first aspect of the present disclosure, it is possible to culture cells and the like in a better environment by increasing the breathability of the film.
The film according to the present disclosure is suitably used for covering a culture plate which cultures, transfers, or stores cells, spheroids, tissues, organs, or the like in wells.
The film according to the present disclosure is usable not only for culture plates but also for dishes (petri dishes), flasks, polystyrene containers, and glass reagent bottles.

## Claims

1. A film adherable to polystyrene on at least one surface thereof.

2. The film according to claim 1, having an adhesion strength to polystyrene of 0.1 to 2.0 N/25 mm on the at least one surface, and an elution amount from the at least one surface to ethyl acetate of 20 g/m² or less at 25 °C.

3. The film according to claim 1 or 2, having an air permeability at 20 °C of 200 sec/100 cc or less.

4. The film according to claim 1, wherein the film contains a styrene elastomer, and
the film has a heat of fusion (ΔH) of 5 to 45 J/g, an arithmetic mean roughness (Ra) of 0.7 µm or less on the at least one surface, and a thickness of 3 to 200 µm.

5. A film roll comprising: a core tube; and the film as recited in claim 4 wound around the core tube.

6. A film storage body for storing the film roll according to claim 5, comprising:
an outlet port from which a film pulled out from the film roll is taken out.

7. A method of protecting a container containing contents by using a film, wherein
the film comprises at least one surface which is an adhesion surface adherable to the container,
the adhesion surface is formed by an adhesion layer which is made of an elastomer containing 50 % by weight or more of at least one of a styrene-conjugated diene block copolymer or a hydrogenated product thereof,
the adhesion surface has an adhesion strength to polystyrene of 0.1 to 2.0 N/25 mm, and
an elution amount from the adhesion surface to ethyl acetate is 20 g/m² or less at 25 °C.
